# EUROPÄISCHE PATENTSCHRIFT

(11) **EP 0 747 364 B1**
(45) Veröffentlichungstag und Bekanntmachung des Hinweises auf die Patenterteilung: **16.04.2003**
(21) Anmeldenummer: 96108408.4
(22) Anmeldetag: 28.05.1996
(51) Int. Cl.: C07D 239/30

(54) **Verfahren zur Herstellung von 2,4,6-Trichlorpyrimidin**
Process for the preparation of 2,4,6-trichloropyrimidine
Procédé pour la préparation de 2,4,6-trichloropyrimidine

(30) Priorität: 09.06.1995 DE 19521036
(43) Veröffentlichungstag der Anmeldung: 11.12.1996
(73) Patentinhaber: DyStar Textilfarben GmbH & Co. Deutschland KG, 65926 Frankfurt am Main (DE)
(72) Erfinder: Hansmann, Wilfried, 51381 Leverkusen (DE); Ehrenberg, Stefan, Dr., 60528 Frankfurt (DE); Stöhr, Frank-Michael, Dr., 51519 Odenthal (DE)

(56) Entgegenhaltungen:
- EP-A- 0 101 561
- EP-A- 0 697 406
- CH-A- 373 045
- DD-A- 221 736
- DE-A- 1 933 784
- CHEMISCHE BERICHTE, Bd.92, 1959 Seiten 2937 - 43, XP000196402 H. BREDERECK ET AL. 'Synthese einiger Uracilyl-pyrazolone'
- JOURNAL OF THE CHEMICAL SOCIETY, 1944 Seiten 678 - 9, XP000196412 J. BADDILEY ET AL. '5-Nitro-4:6-diamino-2-methylpyrimidine and a Modified Procedure for the Preparation of Chloropyrimidines'
- JOURNAL OF THE AMERICAN CHEMICAL SOCIETY, Bd.80, 1958 Seiten 5481 - 3, XP000196413 M.M. ROBISON 'Chlorodehydroxylation of Nitrogen Heterocycles with Phenylphosphonic Dichloride'

## Beschreibung

Die vorliegende Erfindung betrifft ein neues Verfahren zur Herstellung von 2,4,6-Trichlorpyrimidin.

2,4,6-Trichlorpyrimidin kann nach einem Verfahren gemäß Chem. Ber. 92 (1959) 2937 durch Umsetzung von Barbitursäure mit Phosphoroxychlorid (POCl₃) in Gegenwart von Dimethylanilin hergestellt werden. Das so erhaltene 2,4,6-Trichlorpyrimidin wird wäßrig aufgearbeitet. Die Ausbeute, bezogen auf Barbitursäure, beträgt 85 % der Theorie. Dieses Verfahren ist jedoch mit Nachteilen verbunden. So ist beispielsweise für einen großtechnischen Prozeß die wäßrige Aufarbeitung des 2,4,6-Trichlorpyrimidin enthaltenden Reaktionsgemisches umständlich, da in der Regel eine Extraktion der wäßrigen Phase erforderlich ist. Durch die wäßrige Aufarbeitung entstehen zudem große Abwassermengen. Darüber hinaus ist in dem Verfahren eine große Menge des tertiären Amins nötig (etwa 1,8 Mol pro Mol Barbitursäure). Auch das in Beispiel 9 der EP 0 697 406 A1 beschriebene Verfahren arbeitet mit einer großen Menge eines tertiären Amins (2-Methyl-5-ethylpyridin).

Wird eine geringere Menge Dimethylanilin eingesetzt, wie bei dem Verfahren von J. Baddiley et al. J. Chem. Soc. 1944, 678 (etwa 0,8 Mol pro Mol Barbitursäure), sinkt die Ausbeute auf 59 %. Ist für weitere Umsetzungen das 2,4,6-Trichlorpyrimidin als wasserfreies Edukt erforderlich, ist eine anschließende aufwendige Entwässerung nicht zu umgehen. Auch bei Verwendung von Phenylphosphonsäuredichlorid anstelle von Phosphoroxychlorid gemäß dem in M.M.Robison, J. Am. Chem. Soc. 80 (1958) 5481 beschriebenen Verfahren sind diese Nachteile neben geringer Ausbeute zu nennen.

Aus EP 0 101 561 A1 und DE-A 1 933 784 sind Verfahren zur Herstellung von Tetrachlorpyrimidin bekannt, bei denen zunächst Barbitursäure mit Chlor in Phosphoroxychlorid als Lösungsmittel umgesetzt und dann weiter chloriert wird.

Es wurde ein Verfahren zur Herstellung von 2,4,6-Trichlorpyrimidin gefunden, das dadurch gekennzeichnet ist, daß man in einem ersten Reaktionsschritt Barbitursäure in Gegenwart von weniger als 0,5 Mol pro Mol Barbitursäure eines Katalysators mit Phosphoroxychlorid (POCl₃) und anschließend in einem zweiten Reaktionsschritt mit Phosphorpentachlorid (PCl₅) oder mit Phosphortrichlorid (PCI₃) und Chlor, umsetzt, wobei man als Katalysator tertiäre Amine, N-substituierte Carbonsäure- und Sulfonsäureamide, oder N,N-disubstituierte Carbonsäure- und Sulfonsäureamide oder basische Heterocyclen verwendet. Es ist vorteilhaft, wenn man anschließend das gebildete und das nicht umgesetzte Phosphoroxychlorid und das 2,4,6-Trichlorpyrimidin von dem Reaktionsgemisch abtrennt, insbesondere destillativ.

Das insbesondere durch Destillation abgetrennte Phosphoroxychlorid kann beispielsweise dazu verwendet werden, erneut in dem erfindungsgemäßen Verfahren eingesetzt zu werden.

In einer bevorzugten Ausführungsform des erfindungsgemäßen Verfahrens wird der erste Reaktionsschritt bei einer Temperatur von 70 bis 115°C, insbesondere bei der Siedetemperatur bei Normaldruck der Reaktionsmischung und der zweite Reaktionsschritt bei einer Temperatur von 80 bis 120°C, vorzugsweise ebenfalls bei der Siedetemperatur bei Normaldruck der Reaktionsmischung, durchgeführt.

Das Phosphoroxychlorid wird vorzugsweise in einer Menge von 3 bis 6 Mol, insbesondere von 5 bis 6 Mol, bezogen auf 1 Mol Barbitursäure, eingesetzt.

Sowohl größere Mengen als 6 Mol als auch kleinere Mengen als die für die vollständige Umsetzung erforderlichen stöchiometrischen 3 Mol, bezogen auf 1 Mol Barbitursäure, an Phosphoroxychlorid können zwar eingesetzt werden, bieten aber keinen besonderen Vorteil.

Phosphoroxychlorid übernimmt vorzugsweise die Funktion eines Lösungsmittels, so daß das erfindungsgemäße Verfahren vorzugsweise ohne zusätzliche Lösungsmittel durchgeführt werden kann.

Die jeweiligen Reaktionsschritte können jedoch auch in Gegenwart eines inerten Lösungsmittels ablaufen. Ebenfalls vorteilhaft ist es, das Verfahren in Abwesenheit von Wasser durchzuführen. Sowohl im ersten als auch im zweiten Reaktionsschritt, insbesondere aber im zweiten Reaktionsschritt entweicht gebildeter Chlorwasserstoff, der beispielsweise mit Hilfe eines Wäschers aus der Abluft entfernt werden kann.

Im ersten Reaktionsschritt anwesende Katalysatoren sind tertiäre Amine, wie Triethylamin, Tripropylamin, Tri-n-Butylamin, Dimethylanilin, Diethylanilin, N,N-Diethylmethylanilin, N-Ethyl-diisopropylamin, Trioctylamin, Triisobutylamin, 1,8-Bis(Dimethylamino)-naphthalin, N,N-Dimethyl-p-toluidin oder ähnliche, außerdem N-substituierte Carbonsäure- und Sulfonsäureamide oder N,N-disubstituierte Carbonsäure- und Sulfonsäureamide, wie N,N-Dimethylformamid, N,N-Diethylformamid, N,N-Dimethylacetamid, N,N-Diethylacetamid, N-Formylpiperidin, Tetramethylharnstoff, 1-Alkyl-2-pyrrolidone, wie beispielsweise 1-Methyl-2-pyrrolidon (NMP), 1-Octyl-2-pyrrolidon oder 1-Dodecyl-2-pyrrolidon, Dibutylformamid und Methyl-Stearyl-Formamid sowie basische Heterocyclen, wie Pyridin, 2-Methylpyridin, 3-Methylpyridin, 4-Methylpyridin, 2-Ethylpyridin, 4-Ethylpyridin, 2,4-Dimethylpyridin, 2,6-Dimethylpyridin, 3,5-Dimethylpyridin, 5-Ethyl-2-methylpyridin, 2,4,6-Trimethylpyridin, 4-(Dimethylamino)-pyridin, 4-(1-Pyrrolidinyl)-pyridin und Chinolin; natürlich können auch Mischungen der genannten Katalysatoren eingesetzt werden.

Die verwendeten Katalysatoren werden vorzugsweise in einer Menge von weniger als 0,1 Mol pro Mol Barbitursäure eingesetzt. Im zweiten Reaktionsschritt erfolgt die Umsetzung vorzugsweise mit 2,5 bis 3,5 Mol Phosphorpentachlorid oder insbesondere mit 2,5 bis 3,5 Mol Phosphortrichlorid und 2,5 bis 3,5 Mol Chlor pro Mol Barbitursäure. Die Umsetzung des erfindungsgemäßen Verfahrens verläuft insbesondere dann günstig, wenn Phosphortrichlorid und Chlor in stöchiometrischen Mengen eingesetzt werden; d.h. pro Mol Barbitursäure werden 3 Mol Chlor und 3 Mol Phosphortrichlorid eingesetzt. Vorteilhaft ist es, das Phosphortrichlorid ganz oder teilweise vor oder simultan mit dem Chlor zu zudosieren. Vorzugsweise wird Phosphortrichlorid und Chlor simultan der Reaktionsmischung zudosiert. Chlorgas wird dabei vorzugsweise durch ein Gaseinleitungsrohr direkt in die Reaktionsmischung eingeleitet. Besonders vorteilhaft ist es, wenn während der Reaktion kein Chlorüberschuß auftritt.

In einer weiteren bevorzugten Ausführungsform des erfindungsgemäßen Verfahrens wird nach Beendigung des zweiten Reaktionsschrittes das gebildete und das nicht umgesetzte Phosphoroxychlorid in der Reaktionsmischung, d.h. ohne aus der Reaktionsmischung isoliert worden zu sein, mit Barbitursäure und gegebenenfalls unter erneuter Zugabe an Katalysator umgesetzt, wonach sich die Umsetzung mit Phosphorpentachlorid oder mit Phosphortrichlorid und Chlor, anschließt und diese zweistufige Reaktionsfolge gegebenenfalls mehrfach wiederholt.

Die Reaktionsbedingungen dieser gegebenenfalls mehrfach aufeinanderfolgenden zwei Reaktionsschritte entsprechen den erfindungsgemäßen Verfahrensbedingungen.

Dieses Procedere kann mehrmals wiederholt werden, wobei sich die Anzahl der Aufeinanderfolgen und die Menge so hergestellten Produktes in der Regel an den Dimensionen des Reaktors orientiert. Nach der letzten Aufeinanderfolge wird die Reaktionsmischung erfindungsgemäß aufgearbeitet.

Die Ausbeute an 2,4,6-Trichlorpyrimidin, das nach dem erfindungsgemäßen Verfahren erhalten wird, liegt im allgemeinen bei 80 bis 95 % der Theorie, bezogen auf Barbitursäure. Das so erhaltene 2,4,6-Trichlorpyrimidin ist im wesentlichen frei von 2,4,5,6-Tetrachlorpyrimidin sowie von Wasser. Eine Chlorierung an der 5-Position des Pyrimidins wurde unter den erfindungsgemäßen Verfahrensbedingungen nicht beobachtet. Das erfindungsgemäße Verfahren bietet gegenüber den bis jetzt bekannten Verfahren mehrere Vorteile. Neben einer höheren Ausbeute ist das 2,4,6-Trichlorpyrimidin in technisch außerordentlich einfacher Weise aus dem Reaktionsgemisch destillativ in sehr reiner Form zu gewinnen. Zudem fällt bei einer vorzugsweise nichtwäßrigen Aufarbeitung Abwasser erst gar nicht an.

2,4,6-Trichlorpyrimidin ist bekannt als wichtiges Zwischenprodukt für die Herstellung von Reaktivfarbstoffen und als Ausgangsprodukt für die Herstellung von weiteren wichtigen Pyrimidinderivaten wie z.B. 2,4,6-Trifluorpyrimidin.

### Beispiele

### Beispiel 1

500 ml (5,5 Mol) Phosphoroxychlorid wurden in einem 2-I Vierhalskolben vorgelegt. Unter Rühren wurden 128,1 g (1 Mol) Barbitursäure eingetragen. Anschließend wurden 2,5 ml (0,026 Mol) 1-Methyl-2-pyrrolidon (NMP) zugegeben, dann gerührt und unter Rückfluß 2 Stunden zum Sieden erhitzt. Die Reaktionsmischung wurde anschließend auf 80°C abgekühlt und mit 25 ml (0,29 Mol) Phosphortrichlorid versetzt. Anschließend wurden innerhalb von 4 Stunden gleichzeitig 250 ml (2,86 Mol) Phosphortrichlorid und 213,0 g (3 Mol) Chlor zugegeben. Es wurde darauf geachtet, daß während der Reaktion kein Chlorüberschuß auftrat und sobald Chlorgas im Kühler sichtbar wurde, wurde die Chlorzufuhr gedrosselt. Die Reaktionsmischung wurde ständig unter leichtem Rückfluß gehalten. Nach erfolgter Zugabe wurde die Reaktionsmischung bei ca. 110°C mindestens 1 Stunde am Sieden gehalten, bis eine klare Lösung vorlag. Über eine Kolonne wurde anschließend zuerst der größte Teil des Phosphoroxychlorids bei Normaldruck und 110-140°C Sumpftemperatur und dann der Rest bei 20 mbar und 40-80°C Sumpftemperatur, abdestilliert. Bei ca. 95°C und 20 mbar wurden dann 172,0 g (93,8 % der Theorie, bezogen auf Barbitursäure) an 2,4,6-Trichlorpyrimidin abdestilliert. Die Sumpftemperatur wurde dabei von 100°C auf 160°C erhöht.
Gehalt: 99 % (bestimmt durch Gaschromatographie (GC))

### Beispiel 2

25,9 g (0,2 Mol) Barbitursäure, 101,4 ml (1,1 Mol) Phosphoroxychlorid und 0,5 ml (0,005 Mol) 1-Methyl-2-pyrrolidon (NMP) wurden 2 Stunden unter Rückfluß zum Sieden erhitzt. Dann wurden unter Sieden innerhalb 4 Stunden 54,5 ml (0,62 Mol) PCl₃ und 43,0 g (0,61 Mol) Chlor so zugegeben, daß kein Chlorüberschuß auftritt. Unter Sieden wurde 1 Stunde nachgerührt.

Dann wurden 40 g (0,31 Mol) Barbitursäure und 0,8 ml (0,008 Mol) NMP zur Reaktionsmischung gegeben. Die Reaktionsmischung wurde 2 Stunden am Rückfluß gehalten. Dann wurden unter leichtem Sieden innerhalb von 4 Stunden 84,6 ml (0,97 Mol) Phosphortrichlorid und 66,4 g (0,94 Mol) Chlor gleichzeitig so zugegeben, daß kein Chlorüberschuß auftritt. Nach 1 Stunde Nachrühren bei Siedetemperatur wurden 61,7 g (0,48 Mol) Barbitursäure und 1,2 ml (0,012 Mol) NMP zur Reaktionsmischung gegeben. Die Reaktionsmischung wurde 2 Stunden am Sieden gehalten. Dann wurden unter leichtem Sieden innerhalb von 4 Stunden 130,1 ml (1,49 Mol) Phosphortrichlorid und 102,7 g (1,45 Mol) Chlor so zugegeben, daß nie ein Chlorüberschuß auftritt. Nach 1 Stunde Nachrühren bei Siedetemperatur wurde das Phosphoroxychlorid abdestilliert.
Dann wurde im Wasserstrahlvakuum das 2,4,6-Trichlorpyrimidin destilliert. Ausbeute: 149 g (81 % der Theorie, bezogen auf Barbitursäure) Gehalt: 98 % (bestimmt durch GC).

### Beispiel 3

Eine Mischung aus

| | |
|---|---|
| 33,50 kg | (218,5 Mol) Phosphoroxychlorid |
| 5,12 kg | (40,0 Mol) Barbitursäure |
| 1,56 kg | (11,4 Mol) Phosphortrichlorid und |
| 0,30 kg | (1,6 Mol) Tri-n-Butylamin wurde unter Rühren 2 Stunden unter Rückfluß zum Sieden erhitzt. |

Danach wurde auf 80°C abgekühlt und in die Reaktionsmischung, gleichzeitig, innerhalb von 4 Stunden

| | |
|---|---|
| 15,60 kg | (114 Mol) Phosphortrichlorid und |
| 8,51 kg | (120 Mol) Chlor zugegeben, wobei die Reaktionswärme durch |
| | Sieden unter Rückfluß abgeführt wurde (Ansatztemperatur: 100-107°C) |

Nach Beendigung des Chloreinleitens wurde bis zur Beendigung der HCI-Entwicklung zum Rückfluß erhitzt.

Durch die nachfolgende destillative Aufarbeitung wurden

| | |
|---|---|
| 53,20 kg | Phosphoroxychlorid und |
| 6,95 kg | 2,4,6-Trichlorpyrimidin mit einem Gehalt von 97,6 % |

erhalten, was einer Ausbeute von 92,4 % der Theorie, bezogen auf Barbitursäure, entspricht.

### Beispiel 4

500 ml (5,5 Mol) Phosphoroxychlorid wurden in einem 2-I Vierhalskolben vorgelegt. Unter Rühren wurden 128,1 g (1 Mol) Barbitursäure eingetragen. Anschließend wurden 2,5 ml (0,026 Mol) 1-Methyl-2-pyrrolidon (NMP) zugegeben. Die Reaktionsmischung wurde anschließend 7 Stunden bei 80°C gerührt und dann mit 262 ml (3 Mol) Phosphortrichlorid versetzt. Anschließend wurde innerhalb von 4 Stunden 213,0 g (3 Mol) Chlor zugegeben. Es wurde darauf geachtet, daß während der Reaktion kein Chlorüberschuß auftrat und sobald Chlorgas im Kühler sichtbar wurde, wurde die Chlorzufuhr gedrosselt. Die Reaktionsmischung wurde ständig unter leichtem Rückfluß gehalten. Nach erfolgter Zugabe wurde die Reaktionsmischung bei ca. 110°C mindestens 1 Stunde am Sieden gehalten, bis eine klare Lösung vorlag. Über eine Kolonne wurde anschließend zuerst der größte Teil des Phosphoroxychlorids bei Normaldruck und 110-140°C Sumpftemperatur und dann der Rest bei 20 mbar und 40-80°C Sumpftemperatur, abdestilliert. Bei ca. 95°C und 20 mbar wurden dann 172,0 g (93,8 % der Theorie, bezogen auf Barbitursäure) an 2,4,6-Trichlorpyrimidin abdestilliert. Die Sumpftemperatur wurde dabei von 100°C auf 160°C erhöht.

Gehalt: 99 % (bestimmt durch Gaschromatographie (GC)).

## Patentansprüche

1. Verfahren zur Herstellung von 2,4,6-Trichlorpyrimidin, **dadurch gekennzeichnet, daß** man in einem ersten Reaktionsschritt Barbitursäure in Gegenwart von weniger als 0,5 Mol pro Mol Barbitursäure eines Katalysators mit Phosphoroxychlorid und anschließend in einem zweiten Reaktionsschritt mit Phosphorpentachlorid oder mit Phosphortrichlorid und Chlor, umsetzt, wobei man als Katalysator tertiäre Amine, N-substituierte Carbonsäure- und Sulfonsäureamide, oder N,N-disubstituierte Carbonsäure- und Sulfonsäureamide oder basische Heterocyclen verwendet.

2. Verfahren gemäß Anspruch 1, **dadurch gekennzeichnet, daß** man den ersten Reaktionsschritt bei einer Temperatur von 70 bis 115°C und den zweiten Reaktionsschritt bei einer Temperatur von 80 bis 120°C, vorzugsweise bei der Siedetemperatur bei Normaldruck der jeweiligen Reaktionsmischung durchführt.

3. Verfahren gemäß Anspruch 1, **dadurch gekennzeichnet, daß** man im zweiten Reaktionsschritt das Phosphortrichlorid ganz oder teilweise vor oder simultan mit dem Chlor zudosiert.

4. Verfahren gemäß Anspruch 3, **dadurch gekennzeichnet, daß** man für die Umsetzung des zweiten Reaktionsschrittes Phosphortrichlorid und Chlor simultan zur Reaktionsmischung zudosiert.

5. Verfahren gemäß Anspruch 1, **dadurch gekennzeichnet, daß** man als Katalysator Triethylamin, Tributylamin, N,N-Dimethylformamid oder 1-Methyl-2-Pyrrolidon verwendet.

6. Verfahren gemäß Anspruch 1, **dadurch gekennzeichnet, daß** man pro Mol Barbitursäure 2,5 bis 3,5 Mol, insbesondere 3 Mol, Phosphortrichlorid und 2,5 bis 3,5 Mol, insbesondere 3 Mol, Chlor einsetzt.

7. Verfahren gemäß Anspruch 1, **dadurch gekennzeichnet, daß** man nach Beendigung des zweiten Reaktionsschrittes das gebildete und das nicht umgesetzte Phosphoroxychlorid in der Reaktionsmischung mit Barbitursäure und gegebenenfalls unter erneuter Zugabe an Katalysator umsetzt, wonach sich die Umsetzung von Phosphorpentachlorid oder von Phosphortrichlorid und Chlor, anschließt und diese zweistufige Reaktionsfolge gegebenenfalls mehrfach wiederholt.

## Claims

1. Process for preparing 2,4,6-trichloropyrimidine, **characterized in that**, in a first reaction step, barbituric acid is reacted, in the presence of less than 0.5 mol of a catalyst per mol of barbituric acid, with phosphorus oxychloride and subsequently, in a second reaction step, with phosphorus pentachloride or with phosphorus trichloride and chlorine, with tertiary amines, N-substituted carboxamides and sulphonamides or N,N-disubstituted carboxamides and sulphonamides, or basic heterocycles being used as catalyst.

2. Process according to Claim 1, **characterized in that** the first reaction step is carried out at a temperature of from 70 to 115°C and the second reaction step is carried out at a temperature of from 80 to 120°C, preferably at the boiling point of the respective reaction mixture at atmospheric pressure.

3. Process according to Claim 1, **characterized in that** in the second reaction step the phosphorus trichloride is added completely or partially before or simultaneously with the chlorine.

4. Process according to Claim 3, **characterized in that**, for the reaction of the second reaction step, phosphorus trichloride and chlorine are metered simultaneously into the reaction mixture.

5. Process according to Claim 1, **characterized in that** triethylamine, tributylamine, N,N-dimethylformamide or 1-methyl-2-pyrrolidone are used as catalyst.

6. Process according to Claim 1, **characterized in that** from 2.5 to 3.5 mol, in particular 3 mol, of phosphorus trichloride and from 2.5 to 3.5 mol, in particular 3 mol, of chlorine are used per mol of barbituric acid.

7. Process according to Claim 1, **characterized in that** after the second reaction step is complete, the phosphorus oxychloride formed and the unreacted phosphorus oxychloride are reacted in the reaction mixture with barbituric acid, optionally with renewed addition of catalyst, followed by the reaction with phosphorus pentachloride or phosphorus trichloride and chlorine, and this two-stage reaction sequence is repeated a plurality of times if desired.

## Revendications

1. Procédé pour la préparation de 2,4,6-trichloropyrimidine, **caractérisé en ce que**, dans une première étape de réaction, on transforme de l'acide barbiturique, en présence de moins de 0,5 mole par mole d'acide barbiturique d'un catalyseur, avec de l'oxychlorure de phosphore et ensuite, dans une deuxième étape de réaction, avec du pentachlorure de phosphore ou avec du trichlorure de phosphore et du chlore, en utilisant comme catalyseur des amines tertiaires, des amides N-substitués d'acide carboxylique et sulfonique ou des amides N,N-disubstitués d'acide carboxylique et sulfonique ou des hétérocycles basiques.

2. Procédé selon la revendication 1, **caractérisé en ce qu'**on réalise la première étape de réaction à une température de 70 à 115°C et la deuxième étape de réaction à une température de 80 à 120°C, de préférence à la température d'ébullition à la pression normale de chaque mélange réactionnel.

3. Procédé selon la revendication 1, **caractérisé en ce qu'**on ajoute en dosant dans la deuxième étape de réaction le trichlorure de phosphore totalement ou partiellement avant le chlore ou simultanément avec celui-ci.

4. Procédé selon la revendication 3, **caractérisé en ce qu'**on ajoute en dosant, pour la transformation de la deuxième étape de réaction, le trichlorure de phosphore et le chlore simultanément au mélange réactionnel.

5. Procédé selon la revendication 1, **caractérisé en ce qu'**on utilise comme catalyseur de la triéthylamine, de la tributylamine, du N,N-diméthylformamide ou de la 1-méthyl-2-pyrrolidone.

6. Procédé selon la revendication 1, **caractérisé en ce qu'**on utilise, par mole d'acide barbiturique, 2,5 à 3,5 moles, en particulier 3 moles, de trichlorure de phosphore et 2,5 à 3,5 moles, en particulier 3 moles, de chlore.

7. Procédé selon la revendication 1, **caractérisé en ce qu'**on transforme, après la fin de la deuxième étape de réaction, l'oxychlorure de phosphore formé et non transformé dans le mélange réactionnel avec de l'acide barbiturique et, le cas échéant, avec une nouvelle addition de catalyseur, la transformation du pentachlorure de phosphore ou du trichlorure de phosphore et du chlore étant consécutive et cette succession de réactions à deux étapes étant répétée plusieurs fois.
